(19) Europäisches Patentamt
European Patent Office
Office européen des brevets

(11) Publication number: **0 034 829**
**A1**

# (12) EUROPEAN PATENT APPLICATION

(21) Application number: 81101306.9

(22) Date of filing: 23.02.81

(51) Int. Cl.³: C 07 D 211/58
C 08 K 5/34

(30) Priority: 22.02.80 IT 2010480

(43) Date of publication of application:
02.09.81 Bulletin 81/35

(84) Designated Contracting States:
BE CH DE FR GB LI NL

(71) Applicant: Montedison S.p.A.
31, Foro Buonaparte
I-20121 Milan(IT)

(72) Inventor: Roelens, Stefano
17, Viale Andrea Doria
Milan(IT)

(72) Inventor: Di Battista, Piero
36, Via J. Kennedy
San Donato Milanese (Mailand)(IT)

(72) Inventor: Cicchetti, Osvaldo
57, Via Sapri
Milan(IT)

(74) Representative: Weinhold, Peter, Dr. Patentanwälte Dr.
V. Schmied-Kowarzik et al,
Dipl.-Ing. G. Dannenberg Dr. P. Weinhold, Dr. D. Gudel
Dipl.-Ing. S. Schubert Siegfriedstr. 8
D-8000 München 40(DE)

## (54) Tetra-alkyl-4-amino-piperidine containing acids and corresponding salts and use thereof as stabilizers for polymers.

(57) Tetra-alkyl-4-amino piperidine containing acids and corresponding salts having the general formula:

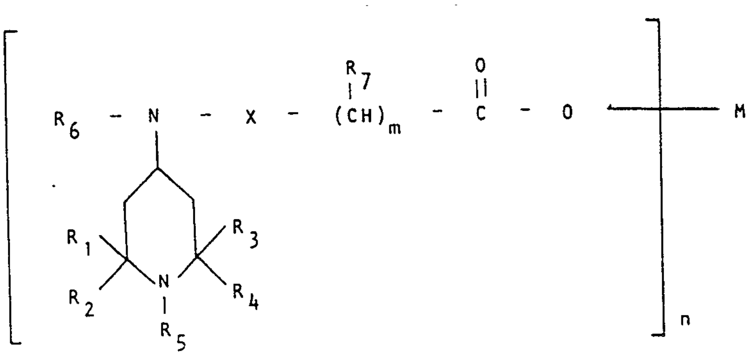

are disclosed. These compounds are useful for stabilizing thermoplastic polymers, in particular polyolefins, against sunlight, heat and oxidation. Polymeric compositions stabilized with said compounds are also disclosed.

EP 0 034 829 A1

TITLE
see front page

0034829



This invention relates to tetra-alkyl-4-amino piperidine containing acids and corresponding salts and to the use thereof for stabilizing thermoplastic polymers against sunlight, heta and oxidation.

It is known that the actinic radiations, particularly in the near ultraviolet region, have a deleterious effect on the appearance and properties of organic polymers. For instance, normally colorless polyesters yellow on exposure to sunlight. The rate of air oxidation of polyolefins is materially accelerated by ultraviolet light. Polystyrene discolors and crack, with accompanying loss of its desirable properties, when exposed to actinic light, etc.

It has been proposed to stabilize polymeric materials against ultraviolet light deterioration by the use of various types of ultraviolet absorbers. For example, in U.S. Patent No. 3,102,107, nickel salts of α-amino-carboxylic acids are disclosed as stabilizers of polyolefins against degradation by light. In U.S. Patent No. 4,007,941 metal salts of N,N-di-substituted beta-alanines are disclosed as UV light stabilizers for synthetic polymeric substances, in particular polyolefins.

The Applicant has now discovered that when the aminic group, in the above specified stabilizers, is tetra-alkyl-substituted 4-amino-piperidine group, the stabilizing activity of the compounds is remarkably and unexpectedly improved.

The stabilizers exhibiting the improved stabilizing properties are, therefore, the tetra-alkyl-substituted 4-amino-piperidine containing acids and metal salts thereof having

...

the general formula:

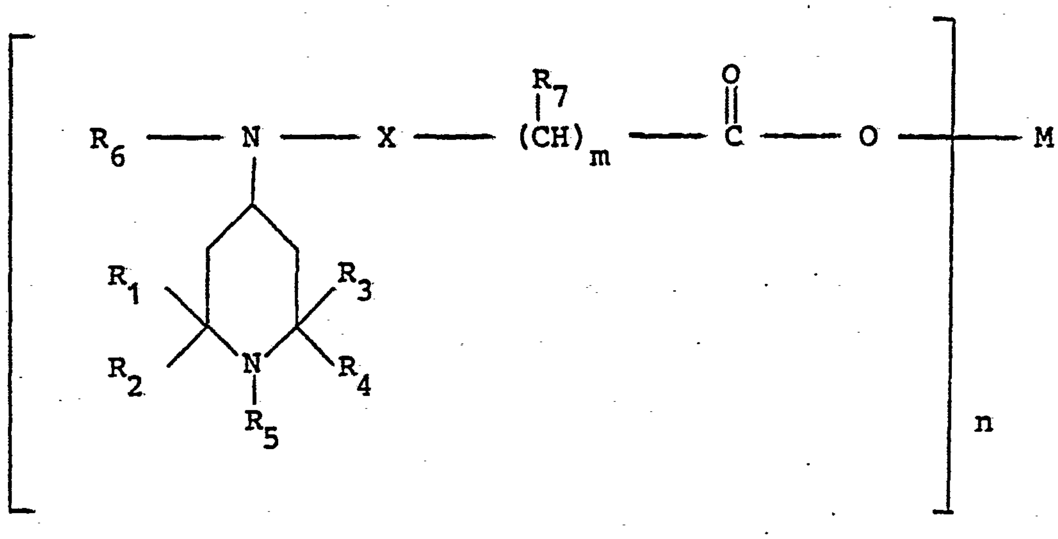

(I)

wherein:

each of $R_1$, $R_2$, $R_3$ and $R_4$, which may be the same or different, is an alkyl radical having 1 to 4 carbon atoms;

$R_5$ is a hydrogen or an alkyl or alkylene radical having 1 to 4 carbon atoms, or a simple or substituted benzyl radical;

X is a $-CH_2-$ or a $-\overset{O}{\overset{\|}{C}}-$ group;

m is an integer from 1 to 12;

$R_7$ is hydrogen or an alkyl radical having 1 to 4 carbon atoms;

$R_6$ is an alkyl-radical having 1 to 12 carbon atoms or a bivalent radical of formula:

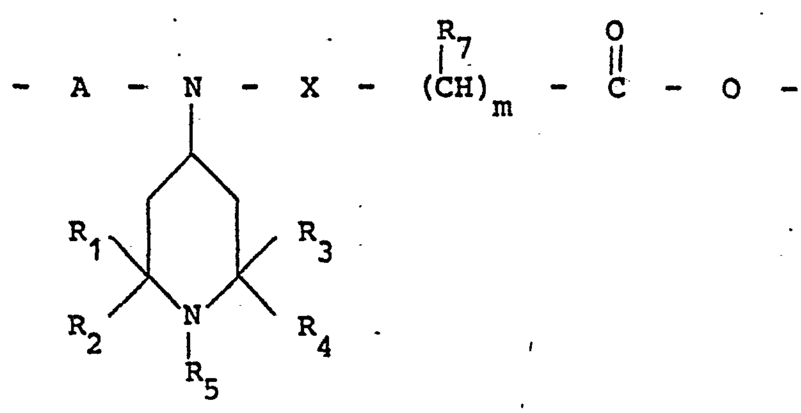

(II)

in which:

A is an alkylene radical having 2 to 10 carbon atoms or a cyclo-alkylene radical having 3 to 12 carbon atoms; and each of $R_1$, $R_2$, $R_3$, $R_4$, $R_5$, $R_7$, X and m has the same meanings as that herein above reported;

n has a value comprised between 1 and 4 and corresponds to the valency of M, when $R_6$ is an alkyl-radical, or it corresponds to a value corresponding to half the valency of M, when $R_6$ is of the above reported general formula (II); and

M is selected from the group consisting of: hydrogen, nickel, chromium, manganese, zinc, aluminum, tin, di-butyl-tin and the radical $Zr\ O^{+2}$.

Group II being attached to the nitrogen atom of compound I via radical A. The substituents $R_1$ - $R_7$ of group II might be different from the corresponding groups of formula I.

The present invention provides, too, compositions based on thermoplastic synthetic polymers, stabilized to ageing and oxidation, containing, as a stabilizer, a tetra-alkyl--substituted 4-amino-piperidine containing acid or corrispond ing metal salt having general formula (I) indicated hereinbefore in an amount sufficient to prevent any degrading action.

The present preferred tetra-alkyl-substituted 4-amino--piperidine containing acids and corresponding metal salts, having general formula (I), for use in the embodiment of the present invention are those in which: $R_1$, $R_2$, $R_3$ and $R_4$ are each a methyl; $R_7$ is hydrogen; X is -CO- or $-CH_2-$; m is an integer from 2 to 6; $R_6$ is an alkyl radical having 1 to 4 carbon atoms or the bivalent radical of general formula (II), wherein A is an alkylene radical having 2 to 6 carbon atoms, and M is hydrogen, nickel, tin or $ZrO^{++}$.

Some examples of compounds having general formula (I)

indicated hereinbefore and which may be used advantageously in the practice of the present invention are: the nickel, zirconile, zinc or manganese salts of one of the following acids:

- 2-methyl-3-[N-(n-butyl)-N-(2,2,6,6-tetramethyl-4-piperydil] amino propionic acid;
- 2-methyl-3-[N-(n-butyl)-N-(1-allyl-2,2,6,6-tetramethyl-4--piperidyl)]-amino propionic acid;
- 3-[N-(n-butyl)-N-(1-benzyl-2,2,6,6-tetramethyl-4-piperidyl)]amino propionic acid;
- 3-[N-cyclohexyl-N-(2,2,6,6-tetramethyl-4-piperidyl)]amino propionic acid;
- 3-[N-isopropyl-N-(2,2,6,6-tetramethyl-4-piperidyl)]amino propionic acid;
- 3-N,N-di-(2,2,6,6-tetramethyl-4-piperidyl)-amino propionic acid;
- 3-N,N-di-(1,2,2,6,6-pentamethyl-4-piperidyl)amino propionic acid;
- 2-methyl-3-N,N-di-(2,2,6,6-tetramethyl-4-piperydil)amino propionic acid;
- 3-[N-(n-butyl)-N-(1,2,2,6,6-pentamethyl-4-piperidyl)]amino propionic acid;
- ethylene-1,2-diamino-N,N'-di-(2,2,6,6-tetramethyl-4-piperidyl)-N,N'-di-3-propionic acid;
- ethylene-1,2-diamino-N,N'-di-(1,2,2,6,6-pentamethyl-4-piperidyl)N,N'-di-3 propionic acid;
- N-mono-succinamide of 4-isopropyl-amino-2,2,6,6-tetramethyl--piperidine;

...

- N-mono-succinamide of 4-cyclohexyl-amino-2,2,6,6-tetramethyl-piperidine;
- N-mono-succinamide of 4-amino-di-(2,2,6,6-tetramethyl-piperidine);
- N-mono-succinamide of 4-benzyl-amino-2,2,6,6-tetramethyl-piperidine;
- 1,6-hexamethylene-diamine-N,N'-di-(2,2,6,6-tetramethyl-4-piperidyl)-bis-mono-succinamide;
- 1,2-ethylene-diamine-N,N'-di-(2,2,6,6-tetramethyl-4-piperidyl)-bis-mono-succinamide;
- N-mono-succinamide-4-butyl-amino-1,2,2,6,6-pentamethyl-piperidine;
- N-mono-succinamide-4-butyl-amino-1-allyl-2,2,6,6-tetramethyl-piperidine;
- N-mono-succinamide-4-butyl-amino-1-benzyl-2,2,6,6-tetramethyl-piperidine;
- N-mono-succinamide-4-amino-1-butyl-2,2,6,6-tetramethyl-piperidine;
- N-mono-glutaramide-4-amino-2,2,6,6-tetramethyl-piperidine,

and the likes.

The above compounds have been listed only for the purpose of illustration of the possible compounds having general formula (I) and all possessing an anti-oxidizing action.

The compounds of general formula (I) can be synthesized according to any conventional method known in the literature.

Thus, for instance, the compounds having general formula (I) in which X is $-CH_2-$, can be synthesized by reacting 4-amino-piperidine with the ester of an unsaturated acid, according to the reaction schema:

...

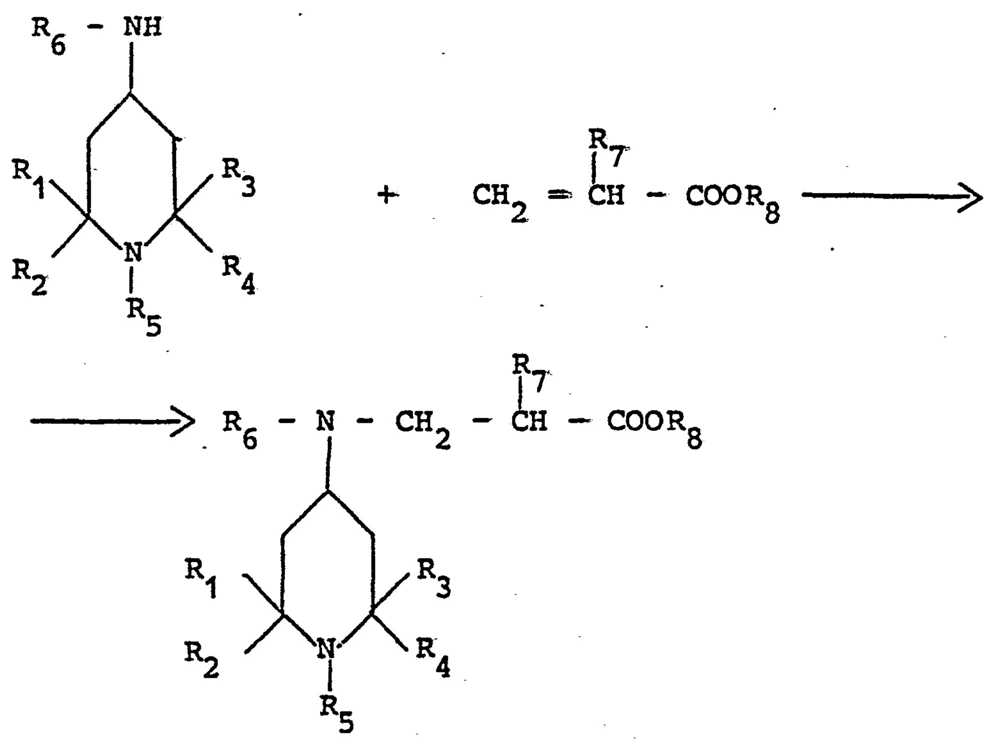

in which $R_8$ is an alkyl radical having 1 to 4 carbon atoms. The obtained ester is subjected to basic hydrolysis and acidification with a mineral acid, and the acid thus obtained or an alkaline salts thereof is reacted with a reactive metal salt, such as for instance $NiCl_2$.

Compounds having general formula (I) in which X is -CO-, can be easily synthesized according to the reaction schema:

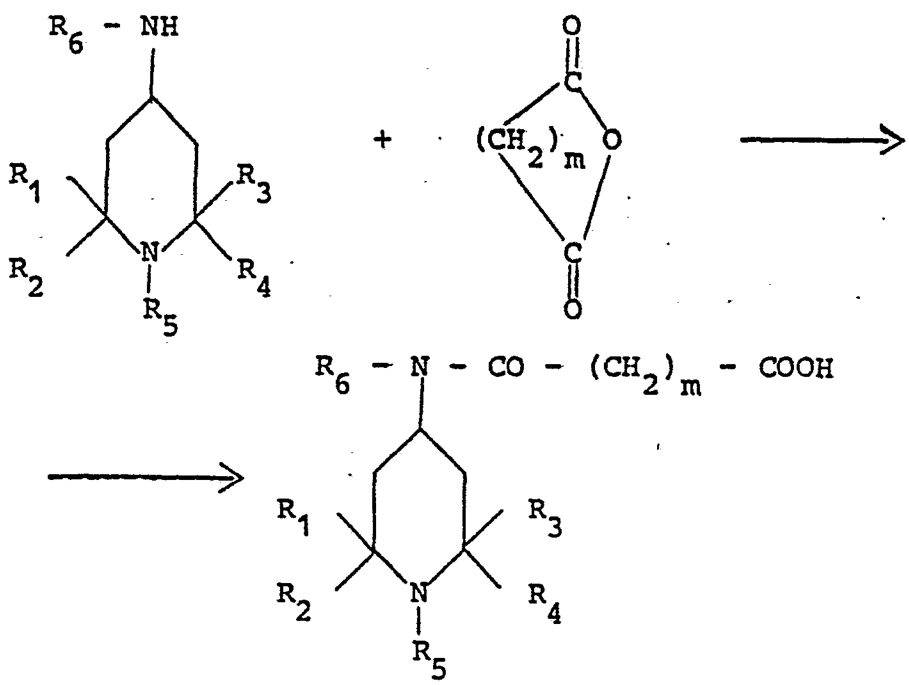

Such synthetic polymers can be used in the form of powder, granules, or shaped articles such as, for example, fibres, films, sheets, etc., and other shaped articles or latexes or foams.

Among the above synthetic polymers, the most suitable one for being used according to this invention, are the polyolefins deriving from monomers having general formula: $R-CH=CH_2$, wherein R is an alkyl or aryl group, or hydrogen.

The present preferred polyolefin is polypropylene consisting prevailingly of isotactic macromolecules and obtained by polymerization of propylene, in the presence of stereospecific catalyst.

The amount of compound, having general formula (I), to be added to the substance to be stabilized, according to this invention, is not critical and may vary in a wide range as function of the type, properties and particular uses of the substance. Generally, said stabilizers can be added in amounts ranging from 0.01 to 5.0% by weight; in practice, however, the effective amount varies as a function of the type of substance to be stabilized. Thus, for example, in the case of polyolefins, the effective amount can range from 0.01% to 2% by weight; in the case of polyvinyl and polyvinylidene chloride, such amount can range from 0.01% to 1% by weight; while, in the case of polyurethanes and polyamides, such amount may range from 0.01% to 5% by weight.

The stabilizer having general formula (I) may be employed either alone or in admixture with other known additive such as: anti-oxidizers, ultraviolet ray absorbers, pigments, fillers, basic nitrogen containing polycondensates, other stabi-

...

and successive treatment of the acid or an alkaline salt thereof with a reactive metal salt, such as for instance $NiCl_2$.

The compounds of general formula (I) may be used as stabilizers of organic materials usually subject to degradation due to the action of heat, oxygen and attinic light.

The materials which can be stabilized with the above--reported compounds, are mainly synthetic organic polymeric substances which include, for instance:

- polyolefins, such as olefin homopolymers, among which high and low density polyethylene, polypropylene, polystyrene, polybutadiene, polyisoprene, and the likes; and copolymers of the olefins with other ethylenically unsaturated monomers, such as ethylene-propylene copolymers, styrene-butene copolymers, styrene-butadiene copolymers, styrene-acrylonitrile copolymers and acrylonitrile-styrene-butadiene copolymers;
- polyvinyl chloride and polyvinylidene chloride, including both the homopolymers and copolymers of vinyl chloride and of vinylidene chloride with each other or each of them with vinyl acetate or with other ethylenically unsaturated monomers;
- polyacetals, such as polyoxymethylene and polyoxyethylene;
- polyesters, such as polyethylene terephtalates;
- polyamides, such as Nylon 6, Nylon 6-6 and Nylon 6-10;
- polyurethanes;
- polycarbonates;
- butadiene-styrene copolymers;
- natural and synthetic rubbers, etc.

...

lizers, etc.

Some examples of such additives are oxi-benzo-triazoles, oxi-benzo-phenones, Ni-stabilizers, metal soaps, phenolic antioxidants, phosphites, thioesters, hydroquinone derivatives, triazine compounds, acyl-amino-phenols, benzyl-phosphonates, etc.

Such additives may be used along with the compounds having general formula (I), according to the present invention, in a ratio by weight ranging from 0.5:1 to 3:1.

The compounds having general formula (I) or the mixture containing said compounds can be incorporated into the synthetic polymer or the substance to be stabilized according to any known procedure and at any stage either before or after polymerization or during the manufacture of the shaped article from the polymer. Thus, for example, it is possible to effect a simple admixing of the additives in powder form, under stirring to the polymer; or the polymer can be mixed together with a solution of the stabilizers in a suitable solvent, which will successively be evaporated; or the stabilizers can be added to the polymer at the end of the polymerization process.

Furthermore, it is possible to get the stabilizing action by applying the stabilizer on the manufactured article, for example, by dipping it into a solution or dispersion of the stabilizer, and then by evaporating the solvent or the dispersant, or by dissolving the stabilizer in a suitable solvent and then by spraying the solution on the surface of the manufactured article.

The following non-limiting examples are given for a more

...

detailed understanding of the present invention and for further enabling those skilled in the art to practice the same.

In the examples, the parts are given by weight, unless otherwise specified.

EXAMPLE No. 1:

Ni(II)bis/N-butyl-N-(2,2,6,6-tetramethyl-4-piperidyl)amino--3-propionate/.

25 g of 4-(n-butyl-amino)-2,2,6,6-tetramethyl-piperidine were dissolved in 50 ml of ethyl alcohol in a 200 ml reactor equipped with a stirrer, a thermometer, a heating jacket and a condenser and connected with a vacuum pump.

12,65 g of methyl-acrylate dissolved in 10 ml of ethyl alcohol and 1.5 ml of acetic acid were added to the above solution.

After 12 hours, the solvent was evaporated under vacuum and the residue was dissolved in $CH_2Cl_2$.

The solution was then washed, in a separator funnel, first with water containing a few drops of NaOH and then with water alone. Thereafter, the solvent was removed and the residue was distilled under vacuum.

30 g of a product (85% yield) were obtained having a boiling point of $133^{o}$-$136^{o}$C, at a pressure of 1 mmHg. On the basis of the nitrogen content, of the molecular weight and of the nuclear magnetic resonance (N.M.R.) spectrum and I.R. spectrum, to the compound was attributed the following structural formula:

...

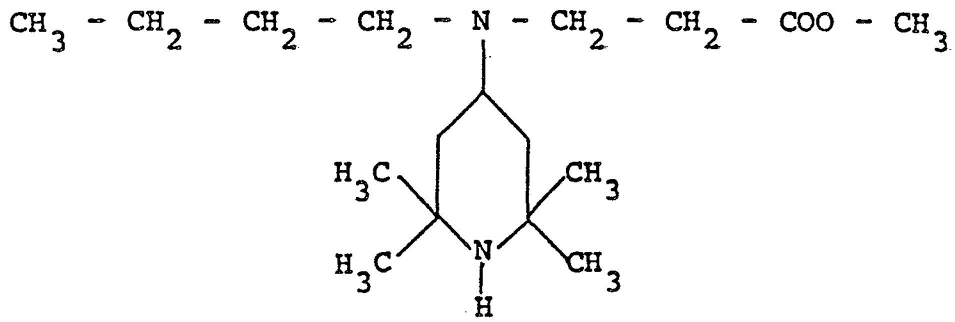

3.8 g of the above methyl N-n-butyl-N-(2,2,6,6-tetramethyl-4--piperidyl)-amino-beta-propionate were dissolved in 100 ml of ethyl-alcohol and then additioned with an aqueous solution consisting of 1.5 ml of water and 0.57 g of NaOH.

This solution was then reflux-heated for 20 hours, after which it was neutralized with HCl.

The solvent was then evaporated. The solid residue was dissolved in ether and the solution filtered through a vacuum pump.

The residue was heat dissolved in $CH_2Cl_2$ and the solution thus obtained was heat filtered. After removal of the solvent from the filtrate, there were obtained 3.3 g (91% yield) of a product which showed a melt point of $192^{\circ}$-$194^{\circ}C$.

On the basis of the nitrogen content, of the molecular weight and of the results of the nuclear magnetic resonance (N.M.R.) and I.R. spectra, to the compound was attributed the following structural formula:

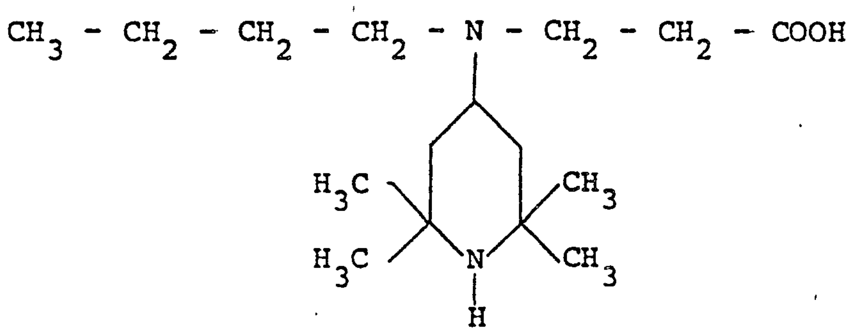

...

5.68 g of the above acid were dissolved in 50 ml of methyl alcohol and the solution was neutralized with a solution of potassium hydroxide in methyl alcohol.

2.38 g of $NiCl_2.6H_2O$, dissolved in 25 ml of methyl alcohol, were added, under continuous stirring, to the above solution. The solution was then maintained for 4 hours under a continuous stirring and was then filtered in order to separate the formed KCl.

The volume of the solution was then concentrated to half its volume, anhydrous methyl alcohol was then added until restoring the original volume and, then, the solution was filtered. These operations of concentrating, restoring the starting volume and of filtering were repeated twice. The solution was then brought to dryness and the residue was dissolved in benzene. The obtained solution was filtered and the filtrate brought to dryness.

A green powder was thereby obtained, which contained 9.27% of Ni corresponding to the formula:

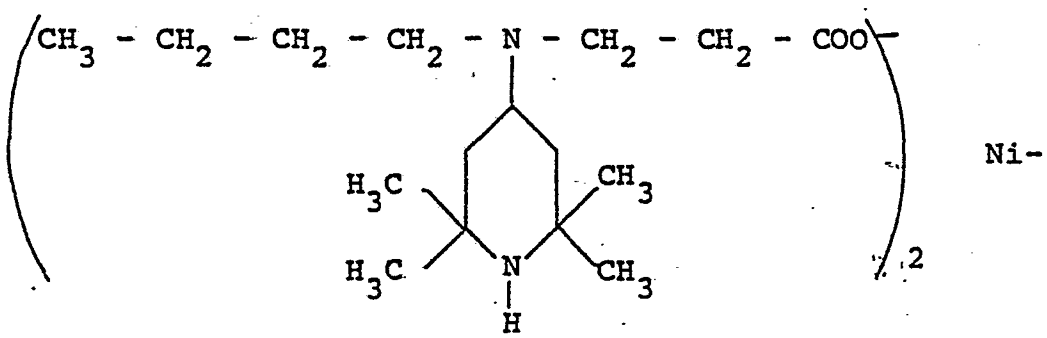

EXAMPLE No. 2:

Zirconyl salt of bis-/N-n-butyl-N-(2,2,6,6-tetramethyl-4-piperidyl)-amino-3-propionic acid/.

It was operated exactly according to the operational condi-

...

tions of example 1, but substituting the Ni-chloride with zirconylchloride.

The resulting product was a white powder having a zirconium content of 13.3%, while the theoretical content is 13.55%.

EXAMPLE No. 3:

The modalities of example 1 were repeated by using hexamethylene-N,N'-di-(2,2,6,6-tetra-methyl-4-piperidyl)-diamine instead of 4(n-butyl-amino)-2,2,6,6-tetramethyl-piperidine.

A green powder was obtained having a Ni content of 9.8%, corresponding to a theoretical Ni content of 9.87%.

By using manganese chloride, zinc chloride or cobalt(ous) chloride instead of Ni-chloride, the corresponding salts were obtained.

EXAMPLE No. 4:

Preparation of metal salts of N-n-butyl-N-(2,2,6,6-tetramethyl-4-piperidyl)mono-succinamide.

21.2 g of 2,2,6,6-tetramethyl-4-n-butyl-amino-piperidine, dissolved in 25 ml of chlorobenzene, were added, under stirring, to 10 g of succinic anhydride, dissolved in 25 ml of chlorobenzene, charged to a 250 ml flask equipped with a stirrer, a thermometer and a reflux-coolant.

The mixture was reflux-heated for 30 minutes and then was cooled down. The solid thus obtained was filtered under vacuum, repeatedly washed with chlorobenzene and finally dried under vacuum.

30 g (yield = 96%) of a white powder, showing a melt point of 210°-214°C, were obtained.

...

One the basis of the elementary analysis and N.M.R. and I.R. spectra, to the product was attributed the following structural formula:

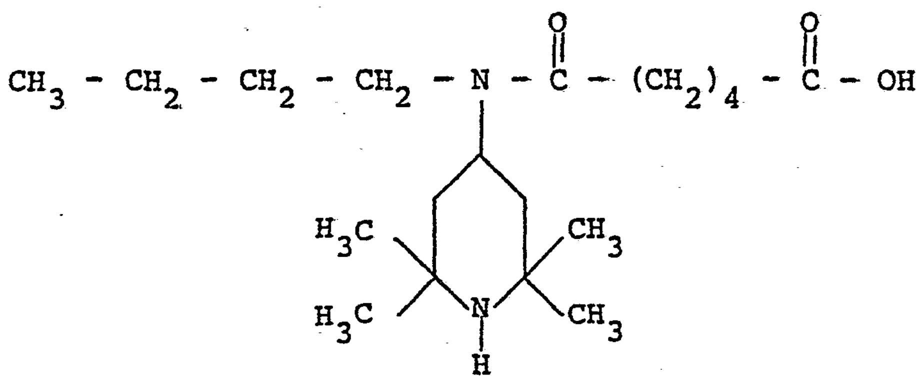

6.25 g of the obtained product were dissolved in 100 ml of methyl alcohol and then neutralized with 1.32 g of potassium hydroxide, dissolved in 50 ml of methyl alcohol.

2.38 g of $NiCl_2.6H_2O$, dissolved in 25 ml of methyl alcohol, were added to the solution. The obtained solution was then kept for 4 hours, under continuous stirring, and filtered in order to separate the formed KCl.

The solution was then concentrated to half its volume, additioned with anhydrous methyl alcohol until restoring the initial volume and then it was filtered.

The concentration, restoring and filtering operations were repeated twice.

The solution was then brought to dryness, the residue dissolved in benzene; the obtained solution was filtered and the filtrate brought in its turn to dryness.

Thereby, 6.51 g (yield = 96%) of a green product, containing 8.24% of Ni, were obtained.

Substituting the $NiCl_2.6H_2O$ with one of the following salts: $ZrOCl_2$, $SnCl_2$, $ZnCl_2$ and $CoCl_2$, the corresponding salts of

...

N-n-butyl-N-(2,2,6,6-tetramethyl-4-piperidyl)-mono-succinamide were obtained.

EXAMPLE No. 5:

Preparation of metal salts of N,N'-hexamethylene-N,N'-di-(2,2,6,6-tetramethyl-4-piperidyl)-bis-mono-succinamide.

It was operated according to the modalities of example 4, by reacting 20 g of succinic anhydride, dissolved in 100 ml of chlorobenzene, with 39.4 g of N,N'-di-(2,2,6,6-tetramethyl-4-piperidyl)-hexamethylene-diamine, dissolved in 90 ml of chlorobenzene.

59 g (yield = 99%) of a white product, in a powdery form, were obtained. On the basis of the elemental analysis and of the N.M.R. and I.R. spectra, the following structural formula was attributed to the obtained product:

$HOOC - (CH_2)_4 - CO - N - (CH_2)_6 - N - CO - (CH_2)_4 - COOH$

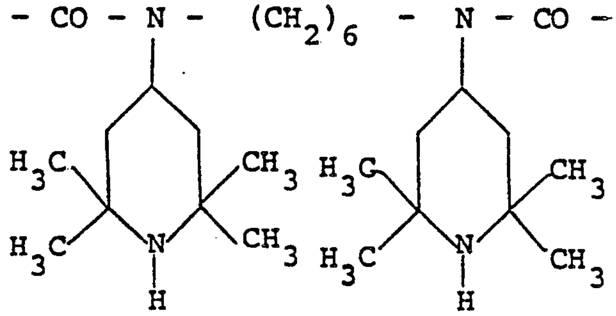

Operating according to the modalities of example 4, the above mono-amide was treated with an equimolar amount of one of the following salts: $NiCl_2 \cdot 6H_2O$; $SnCl_2$, $CoCl_2$, $ZrOCl_2$, and the corresponding salts were obtained.

EXAMPLE No. 6:

Stabilization tests.

200 cc of chloroform, containing dissolved therein 1.5 g of

...

one of the compounds of examples 1 to 5, were added to 300 g of non-stabilized polypropylene, having an intrinsic viscosity, measured in tetraline at 130°C, of 162 cc/g, a residue, after the extraction of the crude polymerizate with n-heptane, of 96.5% and an ashe content of 80 ppm.

Each mixture was stirred for about 6 hours at room temperature, in a rotary evaporator and then dried at 50°C, at 0,01 mm of Hg, for 1 hour.

The additioned powder so obtained was extruded through a Brabender extruder, at 220°C, and granulated. The granules were transformed into films and small plates, at 200°C for 3 minutes, by compression between two square steel plates measuring 20 cm x 20 cm, under a load of 1000 kg.

The films so obtained exhibited an uniform thickness of 50-60 microns and the small plates a thickness of 1 mm.

On the test pieces so prepared, the photo-oxidative stability was determined, by measuring the embrittlement time, considered the time required to cause the rupture of the film, by means of one single bending at 180°C, after exposure to Xenotest 1200, under the following operative conditions:

- temperature of the black panel = 45°C;
- relative humidity = 50%;
- alternating exposure to maximum ultraviolet intensity of the apparatus.

The values of the embrittlement time at the Xenotest are recorded on the following Table.

...

## TABLE

| STABILIZER | Embrittlement time, in hours |
|---|---|
| - Ni(II)bis-[N-butyl-N-(2,2,6,6-tetramethyl-4-piperidyl)-amino,3-propionate] | 1300 |
| - ZrO(II)bis-[N-n-butyl-N-(2,2,6,6-tetramethyl-4-piperidyl)amino 3 propionate] | 900 |
| - Ni(II)bis-hexamethylene-1,6-diamine-N,N'-di-(2,2,6,6-tetramethyl-4-piperidyl)--3-dipropionate | 1860 |
| - 3[N-n-butyl-N-2,2,6,6-tetramethyl-4-piperidyl]amino-propionic acid | 800 |
| - Ni(II)di-3-[N-n-butyl-N-2,2,6,6-tetramethyl-4-piperidyl]amino-propionate | 950 |
| - ZrO(II)di-3-[N-n-butyl-N-2,2,6,6-tetramethyl-4-piperidyl]amino-propionate | 900 |
| - Salt of Ni(II) of 1,6-hexamethylendiamine-N,N'-di-(2,2,6,6-tetramethyl-4-piperidyl)-bis-monosuccinamide | 1650 |
| - Sn(II)salt of 1,6-hexamethylendiamine-N,N'-di-(2,2,6,6-tetramethyl-4-piperidyl)-bis-monosuccinamide | 800 |

## CLAIMS

1) Tetra-alkyl-substituted 4-amino-piperidine containing acids and metal salts thereof having general formula:

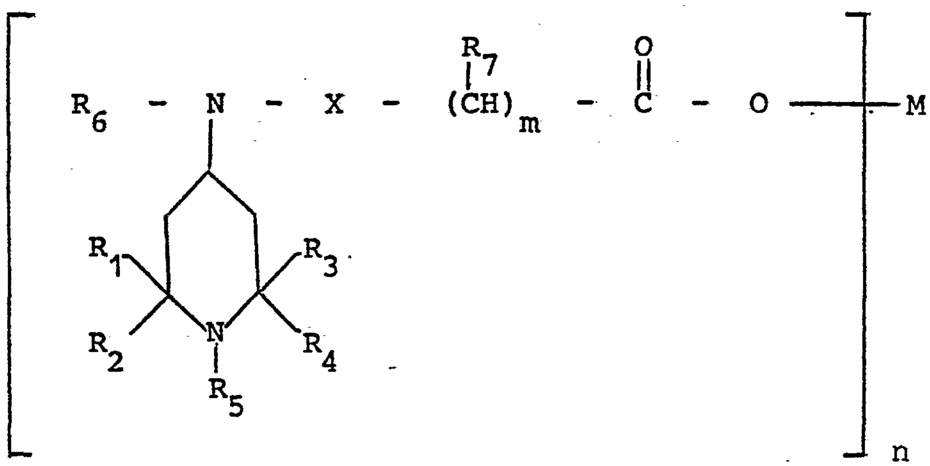

(I)

wherein:

each of $R_1$, $R_2$, $R_3$ and $R_4$, which may be the same or different, is an alkyl radical having 1 to 4 carbon atoms;

$R_5$ is hydrogen or an alkyl or alkylene radical having 1 to 4 carbon atoms, a benzyl radical, or a substituted benzyl radical;

X is either a $-CH_2-$ or $-\overset{O}{\overset{\|}{C}}-$ group;

m is an integer from 1 to 12;

$R_7$ is hydrogen or an alkyl radical having 1 to 4 carbon atoms;

$R_6$ is an alkyl radical having 1 to 12 carbon atoms or a bivalent radical of formula:

...

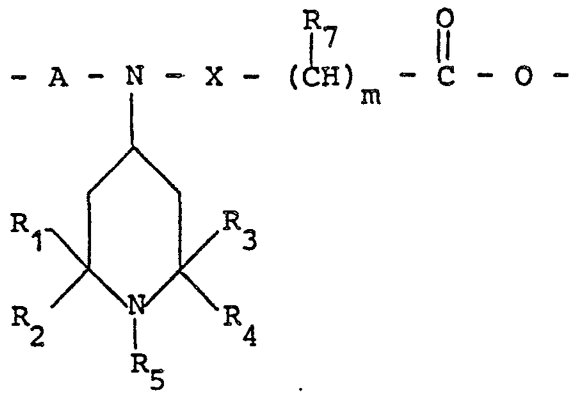

(II)

in which:

A is an alkylene radical having 2 to 10 carbon atoms, or a cyclo-alkylene radical having 3 to 12 carbon atoms; and

each of $R_1$, $R_2$, $R_3$, $R_4$, $R_5$, $R_7$, X and m has the same meanings as that therein above reported;

n has a value comprised between 1 and 4 and corresponds to the valence of M, when $R_6$ is an alkyl radical, or to a value corresponding to half the valency of M, when $R_6$ is of the above reported general formula (II); and

M is selected from the group consisting of: hydrogen, nickel, chromium, manganese, zinc, aluminum, tin, di-butyl-tin and the radical $ZrO^{++}$.

2) Acids and metal salts thereof, having general formula (I), according to claim 1, wherein each of $R_1$, $R_2$, $R_3$ and $R_4$ is a methyl; $R_7$ is hydrogen; X is -CO- or $-CH_2-$; m is an integer from 2 and 6; $R_6$ is an alkyl radical having 1 to 4 carbon atoms, or a bivalent radical of general formula (II), in which A is an alkylene radical having 2 to 6 carbon atoms and M is hydrogen, nickel, tin, or $ZrO^{++}$.

3) Compositions of synthetic thermoplastic polymers, stabi-

...

lized to oxidation, heat and light, having incorporated therein, in an amount sufficient to prevent any degradation of the polymer, a tetra-alkyl-substituted 4-amino--piperidine containing acid or a metal salt thereof, having general formula:

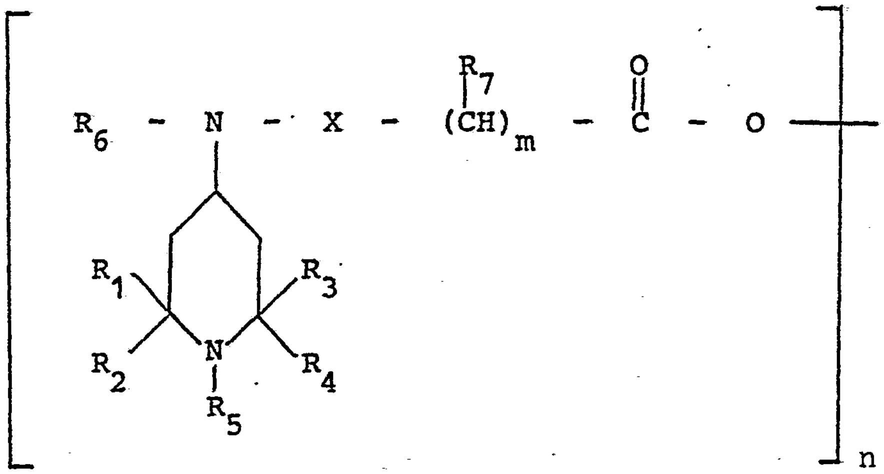

(I)

wherein:

each of $R_1$, $R_2$, $R_3$ and $R_4$, which may be the same or different, is an alkyl radical having 1 to 4 carbon atoms;

$R_5$ is hydrogen or an alkyl or alkylene radical having 1 to 4 carbon atoms, a benzyl radical or a substituted benzyl radical;

X is either a $-CH_2-$ or $-\overset{O}{\overset{\|}{C}}-$ group;

m is an integer from 1 to 12;

$R_7$ is hydrogen or an alkyl radical having 1 to 4 carbon atoms;

$R_6$ is an alkyl radical having 1 to 12 carbon atoms or a bivalent radical of formula:

...

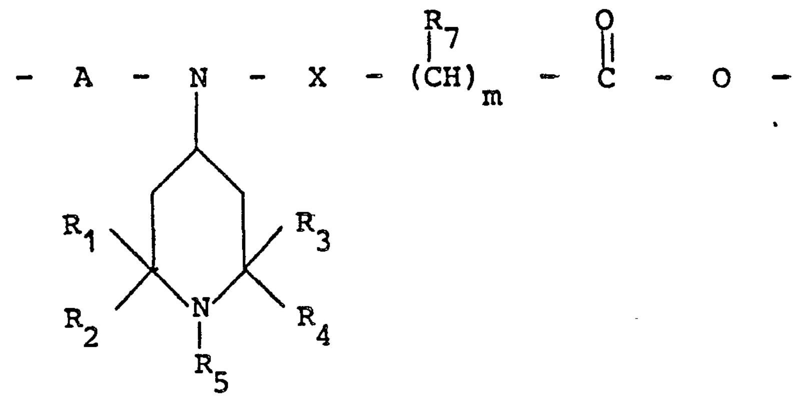

(II)

in which:

A is an alkylene radical having 2 to 10 carbon atoms, or a cyclo-alkylene radical having 3 to 12 carbon atoms; and each of $R_1$, $R_2$, $R_3$, $R_4$, $R_5$, $R_7$, X and m has the same meanings as that therein above reported;

n has a value comprised between 1 and 4 and corresponds to the valence of M, when $R_6$ is an alkyl radical, or to a value corresponding to half the valency of M, when $R_6$ is of the above reported general formula (II); and

M is selected from the group consisting of: hydrogen, nickel, chromium, manganese, zinc, aluminum, tin, di-butyl-tin and the radical $ZrO^{++}$.

4) Compositions according to claim 3, wherein the acid or the salt thereof having general formula (I) is present in an amount ranging from 0.01% to 5% by weight on the weight of the polymer.

5) Compositions according to claim 3, wherein the synthetic thermoplastic polymer is a polyolefin.

6) Compositions according to claim 5, wherein the polyolefin is polypropylene consisting prevailingly of isotactic macromolecules.

7) Compositions according to claim 5, wherein the acid or

...

the salt thereof having general formula (I) is present in an amount ranging from 0.01% to 2% by weight on the weight of the polyolefin.

8) Compositions according to claim 3, wherein the thermoplastic synthetic polymer is polyvinylchloride or polyvinylidene chloride.

9) Compositions according to claim 8, in which the acid or the salt thereof having general formula (I) is present in an amount ranging from 0.01 to 1% by weight on the weight of the polymer.

10) Compositions according to claim 3, in which the thermoplastic synthetic polymer is a polyurethane or a polyamide.

11) Compositions according to claim 10, in which the acid or the salt thereof having general formula (I) is present in an amount ranging from 0.01 to 5% by weight on the weight of the polymer.

12) Compositions according to claim 3, in which the acid or the salt thereof having general formula (I) is employed in admixture with other known additives selected from the group consisting of: anti-oxidants, U.V. radiation absorbers, pigments, fillers, basic nitrogen containing polycondensates and auxiliary stabilizers.

13) Compositions according to claim 12, in which the ratio by weight of the known additives to the acid or the salt thereof having general formula (I) is from 0.5:1 and 3:1.

0034829

European Patent Office

# EUROPEAN SEARCH REPORT

Application number
EP 81101306.9

| DOCUMENTS CONSIDERED TO BE RELEVANT | | | CLASSIFICATION OF THE APPLICATION (Int. Cl.3) |
|---|---|---|---|
| Category | Citation of document with indication, where appropriate, of relevant passages | Relevant to claim | |
| | GB - A - 1 492 494 (CIBA-GEIGY)<br>+ Claims 1,9-11,13,22,37,39 +<br>-- | 1,3-5, 7-10, 12 | C 07 D 211/58<br>C 08 K 5/34 |
| | US - A - 3 684 765 (MATSUI)<br>+ Claims 1-5 +<br>-- | 1,3-5, 8,10 | |
| | GB - A - 1 518 924 (BAYER)<br>+ Claims 1,9,11,12 +<br>---- | 1,3,4, 9 | |
| | | | TECHNICAL FIELDS SEARCHED (Int. Cl.3) |
| | | | C 07 D 211/00<br>C 08 K |
| | | | CATEGORY OF CITED DOCUMENTS<br>X: particularly relevant<br>A: technological background<br>O: non-written disclosure<br>P: intermediate document<br>T: theory or principle underlying the invention<br>E: conflicting application<br>D: document cited in the application<br>L: citation for other reasons |
| X | The present search report has been drawn up for all claims | | &: member of the same patent family, corresponding document |

| Place of search | Date of completion of the search | Examiner |
|---|---|---|
| VIENNA | 13-05-1981 | HOCHHAUSER |

EPO Form 1503.1 06.78